Europäisches Patentamt

European Patent Office

Office européen des brevets

(19)

(11) Publication number: 0 290 278
A1

# EUROPEAN PATENT APPLICATION

(12)

(21) Application number: 88304137.8

(22) Date of filing: 06.05.88

(51) Int. Cl.⁴: **A 61 B 5/00**
G 01 N 21/31

(30) Priority: 08.05.87 JP 110470/87

(43) Date of publication of application:
09.11.88 Bulletin 88/45

(84) Designated Contracting States: DE GB

(71) Applicant: HAMAMATSU PHOTONICS K.K.
1126-1 Ichino-cho Hamamatsu-shi
Shizuoka-ken (JP)

(72) Inventor: Suzuki, Susumu
Hamatsu Photonics K.K. No. 1126-1 Ichino-cho
Hamamatsu-shi Shizuoka (JP)

Yagi, Sumio
Hamatsu Photonics K.K. No. 1126-1 Ichino-cho
Hamamatsu-shi Shizuoka (JP)

Hakamata, Naotoshi
Hamatsu Photonics K.K. No. 1126-1 Ichino-cho
Hamamatsu-shi Shizuoka (JP)

Ozaki, Takeo
Hamatsu Photonics K.K. No. 1126-1 Ichino-cho
Hamamatsu-shi Shizuoka (JP)

(74) Representative: Rackham, Stephen Neil et al
GILL JENNINGS & EVERY 53-64 Chancery Lane
London WC2A 1HN (GB)

(54) Examination apparatus for measuring oxygenation.

(57) An examination apparatus (1) for measuring oxygenation can examine a plurality of measurement locations at the same time. The plurality of measurement locations may be a plurality of objects, persons or a plurality of parts of the same object or person. The examination apparatus comprises light source means (LD1m-LD4m, 3-1 to 3-m) for emitting electromagnetic radiation at a number of different wavelengths, a plurality of illumination-side fixtures (6-1 to 6-m), a plurality of detection side fixtures (7-1 to 7-m), transmitted light detection means (4), and a computer system (2) for controlling the apparatus and analysing the results. The light source means typically emit near infrared light radiation of four different wavelengths. Transmitted radiation from the plurality of measurement locations are sequentially measured with a time-sharing method.

FIG. 1

EP 0 290 278 A1

## Description

## Examination Apparatus For Measuring Oxygenation

The present invention relates to the apparatus for measuring the oxygen concentration in objects such as organs, e.g. the cerebral tissues, of a human body or animals, especially relates to the apparatus for measuring the oxygenation of hemoglobin in blood and that of cytochrome in cells by detecting their effect on electromagnetic radiation.

In general, in diagnosing the function of the body organ, e.g. the cerebral tissues, it is a fundamental and important parameter whether the oxygen quantity in the body organ is sufficient and it is suitably used. Supplying body organs with sufficient quantity of oxygen is indispensable for the growth ability of foetuses and new-born infants. If the supply of oxygen is insufficient, the death rates of foetuses and new-born infants are high, and even if they live serious problems in body organs may remain as a consequence. The insufficiency of oxygen affects every body organ, especially causes a serious damage in the cerebral tissues.

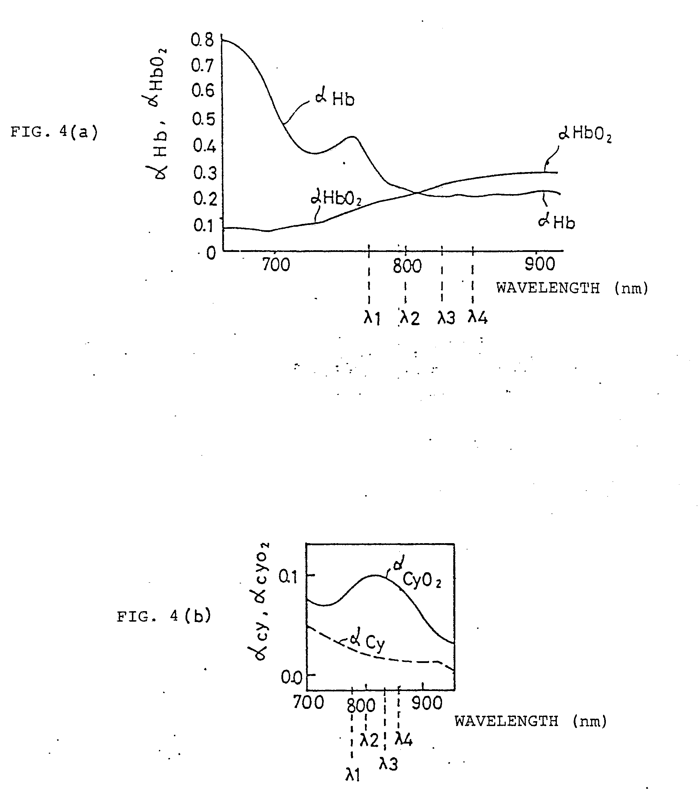

To examine the oxygen quantity in body organs readily and at the early stage of illness, an examination apparatus disclosed in US-A-4,281,645 has been developed. In this kind of examination apparatus, the variation of oxygen quantity in body organs, especially in the brain is measured through the absorption spectrum of near infrared light in which the absorption is caused by the hemoglobin which is an oxygen-carrying medium in blood and the cytochrome a, $a_3$ which performs oxydation-reduction reaction in cells. As shown in Fig. 4(a), the absorption spectra of near infrared light (700 to 1300 nm), $\alpha_{HbO2}$ and $\alpha_{Hb}$ by oxygenated hemoglobin (HbO$_2$) and disoxygenated hemoglobin (Hb), respectively, are different from each other. And as shown in Fig. 4(b), the absorption spectra of that, $\alpha_{CyO2}$ and $\alpha_{Cy}$ by oxidized cytochrome a, $a_3$ (CyO$_2$) and reduced cytochrome a, $a_3$ (Cy), respectively, are different from each other. This examination apparatus utilizes the above-described absorption spectra of near infrared light. Four near infrared light rays with different wavelengths, $\lambda_1, \lambda_2, \lambda_3$ and $\lambda_4$ (e.g. 775 nm, 800 nm, 825 nm and 850 nm) are applied to one side of the patient's head with a time-sharing method and the transmission light rays from the opposite side of the head are in turn detected. By processing these four detected results with the prescribed calculation program, four unknown quantities, i.e. the density variations of oxygenated hemoglobin (HbO$_2$), disoxygenated hemoglobin (Hb), oxidized cytochrome a, $a_3$ (CyO$_2$) and reduced cytochrome a, $a_3$ (Cy) are calculated and being based on these parameters, for example the variation of a cerebral oxygen quantity is obtained.

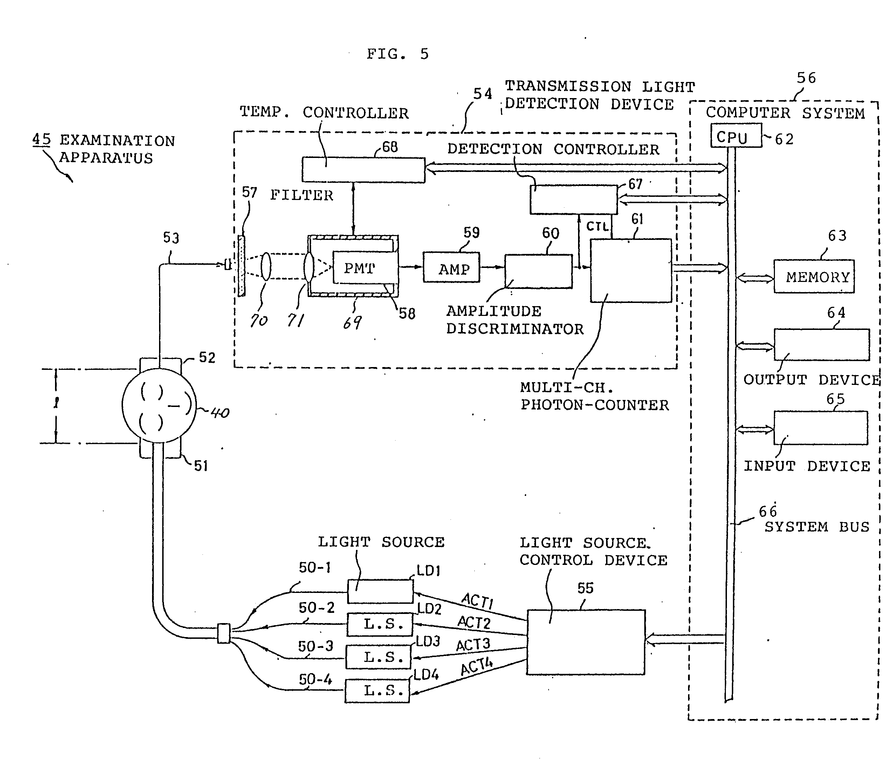

Fig. 5 shows a system outline of the above-described conventional examination apparatus 45. The conventional examination apparatus 45 includes; light sources such as laser diodes LD1 to LD4 which emit four near infrared light rays with different wavelengths of $\lambda_1, \lambda_2, \lambda_3$ and $\lambda_4$, respectively; a light source control device 55 which controls output timing of the light sources LD1 to LD4; optical fibers 50-1 to 50-4 which introduces near infrared light rays emitted by the light sources LD1 to LD4 to a patient's head 40; an illumination-side fixture 51 which bundles and holds end portions of the optical fibers 50-1 to 50-4; a detection-side fixture 52 which is fitted to the prescribed position of the opposite side of the patient's head 40; a optical fiber 53 which is held by the detection-side fixture 52 and introduces transmitted near infrared light from the patient's head 40; a transmission light detection device 54 which measures transmission quantity of near infrared light by counting photons of near infrared light introduced by the optical fiber 53; and a computer system 56 which controls the total examination apparatus and determines the variation of oxygen quantity in cerebral tissues being based on the transmission quantity of near infrared light.

The computer system 56 is equipped with a processor 62, a memory 63, output devices 64 such as a display and a printer, and an input device 65 such as a keyboard, and these devices are connected each other by a system bus 66. The light source control device 55 and the transmission light detection device 54 are connected to the system bus 66 as external I/O's.

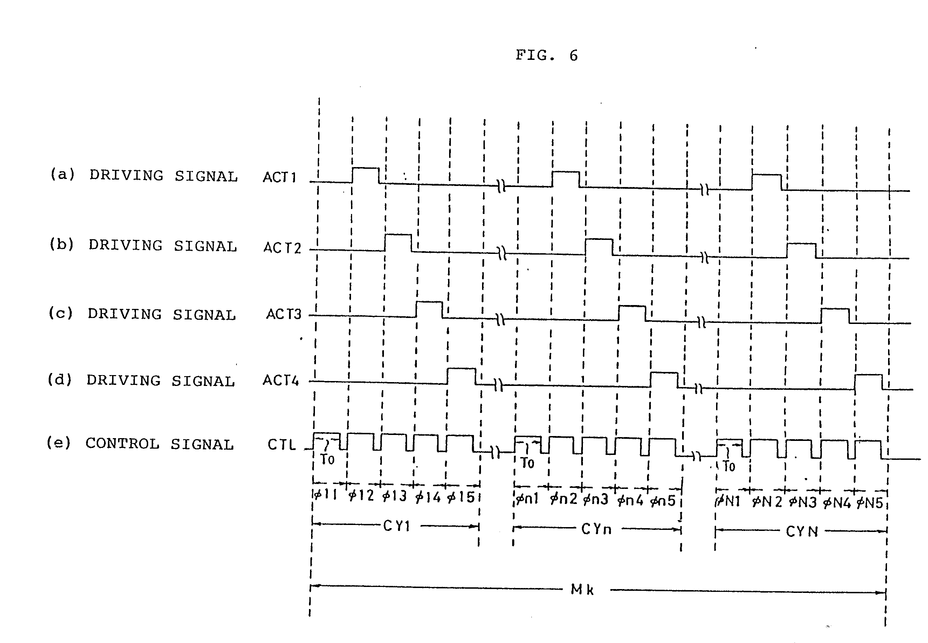

The light source control device 55 drives the light sources LD1 to LD4 by respective driving signals ACT1 to ACT4 as shown in Fig. 6(a) to 6(d) according to instructions from the computer system 56. As shown in Fig. 6 one measuring period $M_k$ (k = 1, 2, .....) consists of N cycles of CYl to CYn. At a phase $\Phi n1$ in an arbitrary cycle CYn, no light source of LD1 to LD4 is driven and therefore the patient's head 40 is not illuminated by the near infrared light from the light sources LD1 to LD4. At the phase $\Phi n2$ the light source LD1 is driven and the near infrared light with the wavelength of for example 775 nm is emitted from it. In the same manner, at the phase $\Phi n3$ the light source LD2 is driven and the near infrared light with the wavelength of for example 800 nm is emitted from it; at the phase $\Phi n4$ the light source LD3 is driven and the near infrared light with the wavelength of for example 825 nm is emitted from it; and at the phase $\Phi n5$ the light source LD4 is driven and the near infrared light with the wavelength of for example 850 nm is emitted from it. In this manner the light source control device 55 drives the light sources LD1 to LD4 sequentially with a time-sharing method.

The transmission light detection device 54 is equipped with a filter 57 which adjusts the quantity of near infrared light outputted from the optical fiber 53; lenses 70 and 71; a photomultiplier tube 58 which converts the light from the filter 57 into pulse current and outputs it; an amplifier 59 which amplifies the pulse current from the photomultiplier tube 58; an amplitude discriminator 60 which eliminates the pulse current from the amplifier 59 whose amplitude is smaller than the prescribed threshold value; a multi-channel photon-counter 61 which detects photon frequency in every channel; for example a detection controller 67 which controls detection periods of the multi-channel photon-counter 61; a temperature controller 68 which controls the temperature of a cooler 69 containing the photomultiplier tube 58.

In use of the above-described examination apparatus, the illumination-side fixture and the detection-side fixture are firmly fitted to the prescribed positions of the patient's head 40 by using a tape or the like. After that, the light sources LD1 to LD4 are driven by the light source control device 55 as shown in Fig. 6(a) to 6(d), respectively, so that the four near infrared light rays with different wavelengths are emitted from the light sources LD1 to LD4 sequentially with the time-sharing method, and the light rays are introduced by the optical fibers 50-1 to 50-4 to the patient's head 40. As bones and soft tissues in the patient's head 40 are transparent to the near infrared light, the near infrared light is partially absorbed mainly by hemoglobin in blood and cytochrome a, $a_3$ in cells and outputted to the optical fiber 53. And the optical fiber 53 introduces the light to the transmission light detection device 54. At the phase $\Phi n1$ no light source of LD1 to LD4 is driven, so that the transmission light detection device 54 does not receive the transmission light originally emitted from the light sources LD1 to LD4. At this phase the transmission light detection device 54 detects dark light.

The photomultiplier tube 58 in the transmission light detection device 54 is the one for photon-counting which has high sensitivity and operates at high response speed. The output pulse current from the photomultiplier tube 58 is sent to the amplitude discriminator 60 through the amplifier 59. The amplitude discriminator 60 eliminates the noise component whose amplitude is smaller than the prescribed amplitude threshold and sends only the signal pulse to the multi-channel photon-counter 61. The multi-channel photon-counter 61 detects photon number only in the periods $T_0$ which is made synchronized with the driving signals ACT1 to ACT4 for the respective light sources LD1 to LD4 as shown in Fig. 6(a) to (d) by a control signal CTL as shown in Fig. 6(e) from the detection controller 67, and counts detected photon number of every light with each wavelength sent from the optical fiber 53. The transmission data of every near infrared light with each wavelength are obtained through the above-described procedure.

That is, as shown in Fig. 6(a) to (e), at the phase $\Phi n1$ in the cycle CYn of light source control device 55 no light source of LD1 to LD4 is driven, therefore the dark light data $\underline{d}$ are counted by the transmission light detection device 54. At the phases $\Phi n2$ to $\Phi n5$ the light sources $LD\overline{1}$ to LD4 are sequentially driven with the time-sharing method and the transmission light detection device 54 sequentially counts the transmission data $T_{\lambda 1}$, $T_{\lambda 2}$, $T_{\lambda 3}$ and $T_{\lambda 4}$ of the respective near infrared light rays with different wavelengths $\lambda_1$, $\lambda_2$, $\lambda_3$ and $\lambda_4$.

The counting of the dark light data $\underline{d}$ and the transmission data $T_{\lambda 1}$, $T_{\lambda 2}$, $T_{\lambda 3}$ and $T_{\lambda 4}$ which is sequentially performed in the cycle CYn, is continued N times from CY1 to CYn. That is, one measuring period $M_k$ ($k = 1, 2, .....$) includes N cycles. A concrete example is as follows; if one cycle is 200 $\mu$sec and N is 10000, the measuring period $M_k$ becomes 2 sec. At the time of finishing of one measuring period $M_k$, the counting result of the dark light data D

$$( \ = \ \sum_{n=1}^{N} d/CYn )$$

and the counting results of the transmission data $T_{\lambda 1}$, $T_{\lambda 2}$, $T_{\lambda 3}$ and $T_{\lambda 4}$ ( $=$

$$\sum_{n=1}^{N} t_{\lambda j} \ / \ CYn )$$

are transferred to the computer system 56 and stored in the memory 63.

The processor 62 performs the subtraction of the dark light component by using the combination of the transmission data and the dark data $(T_{\lambda 1}, T_{\lambda 2}, T_{\lambda 3}, T_{\lambda 4}, D)M_k$ being stored in the memory 63 after one measuring period $M_k$ and the combination of those $(T_{\lambda 1}, T_{\lambda 2}, T_{\lambda 3}, T_{\lambda 4}, D)M_0$ at the start of measuring, and calculates the variation rates of the transmission light $\Delta T_{\lambda 1}$, $\Delta T_{\lambda 2}$, $\Delta T_{\lambda 3}$ and $\Delta T_{\lambda 4}$. That is, the variation rates of the transmission light $\Delta T_{\lambda 1}$, $\Delta T_{\lambda 2}$, $\Delta T_{\lambda 3}$ and $\Delta T_{\lambda 4}$ are calculated as:

$\Delta T_{\lambda j} = \log \ [(T_{\lambda j}-D)_{Mk}|(T_{\lambda j}-D)_{Mo}] \ (j = 1 \ \text{to} \ 4).$     (1)

The use of logarithm in the above calculation of $\Delta T_{\lambda j}$ is to express the variation as an optical density.

Using the above-calculated variation rates of the transmission light $\Delta T_{\lambda 1}$, $\Delta T_{\lambda 2}$, $\Delta T_{\lambda 3}$ and $\Delta T_{\lambda 4}$, density variations of oxygenated hemoglobin ($HbO_2$), disoxygenated hemoglobin (Hb), oxidized cytochrome a, $a_3$ ($CyO_2$) and reduced cytochrome a, $a_3$ which are expressed as $\Delta X_{HbO_2}$ $\Delta X_{Hb}$, $\Delta X_{CyO_2}$ and $\Delta X_{Cy}$, respectively, can be determined. That is, each of density variations of $\Delta X_{HbO_2}$ $\Delta X_{Hb}$, $\Delta X_{CyO_2}$ and $\Delta X_{Cy}$ is calculated as:

$$\Delta X_i = \sum_{j=1}^{4} (\alpha_{ij})^{-1} \Delta T_{\lambda j} / \ell \qquad \cdots\cdots\cdots (2)$$

where $\alpha_{ij}$ is an absorption coefficient of each component i (HbO$_2$, Hb, CyO$_2$, Cy) for each Wavelength $\lambda_j$ ($\lambda_1$, $\lambda_2$, $\lambda_3$, $\lambda_4$) and is predetermined from Fig. 4(a) and (b), and $\ell$ is the length of the patient's head 40 along the travelling direction of the near infrared light.

As the above-detected density variation components, $\Delta X_{HbO_2}$, $\Delta X_{Hb}$, $\Delta X_{CyO_2}$ and $\Delta X_{Cy}$, reflect the variation of oxygen quantity in the brain, the variation of oxygen quantity in the brain can be known by outputting these detected results from the output device 64 and the diagnosis is made being based on these results.

By the way, it is sometimes desired in the foregoing examination apparatus to examine not only one object person but also plural object persons at the same time, or to examine not only one part of an object person but also plural parts substantially at the same time, to improve the efficiency of the examination system. But the foregoing conventional examination apparatus could not satisfy the above requirements, because merely one pair of the illumination-side fixture 51 and the detection-side fixture 52 is equipped in the conventional examination apparatus.

According to this invention an examination apparatus for measuring the oxygenation of objects by electromagnetic wave transmission spectrophotometry, comprising:

light source means for sequentially emitting electromagnetic radiation at a number of different wavelengths;

transmitted light detection means for detecting electromagnetic radiation transmitted through an object and outputting transmission light data; and,

a computer system for controlling the light source means and the transmitted light detection means and for receiving the transmission light data from the transmission light detection means and calculating the oxygenation parameters;

is characterised in that the examination apparatus also includes:

a plurality of illumination-side fixtures for applying electromagnetic radiation emitted by the light source means to a plurality of measurement locations; and,

a plurality of detection-side fixtures for receiving electromagnetic radiation transmitted through the plurality of measurement locations and coupling the transmitted electromagnetic radiation to the transmitted light detection means;

the computer system calculating the oxygenation parameters for each of the plurality of measurement locations.

An advantage of the present invention is that it provides an examination apparatus with a high system efficiency which can examine a plurality of measurement locations at substantially the same time.

Particular embodiments of examination apparatus in accordance with this invention will now be described with reference to the accompanying drawings, in which:-

Figure 1 is a block diagram of an examination apparatus according to a first embodiment of the present invention;

Figure 2 shows timing-charts to explain the oepration of the examination apparatus shown in Figure 1, where Figure 2(a) to (f) are time-charts of a light source control signal LAC1, a driving signal ACTX1, a light source control signal LACm and a driving signal ACTXm, respectively;

Figure 3 is a block diagram of an examination apparatus according to a second embodiment of the present invention;

Fig. 4(a) and (b) are graphs showing absorption spectra of hemoglobin and cytochrome, respectively;

Fig. 5 is a block diagram showing a system constitution of a conventional examination apparatus; and

Fig. 6(a) to (e) are time-charts of driving signals ACT1 to ACT4 and a control signal CTL, respectively.

Embodiments of the present invention are hereafter described with referring to the attached drawings.

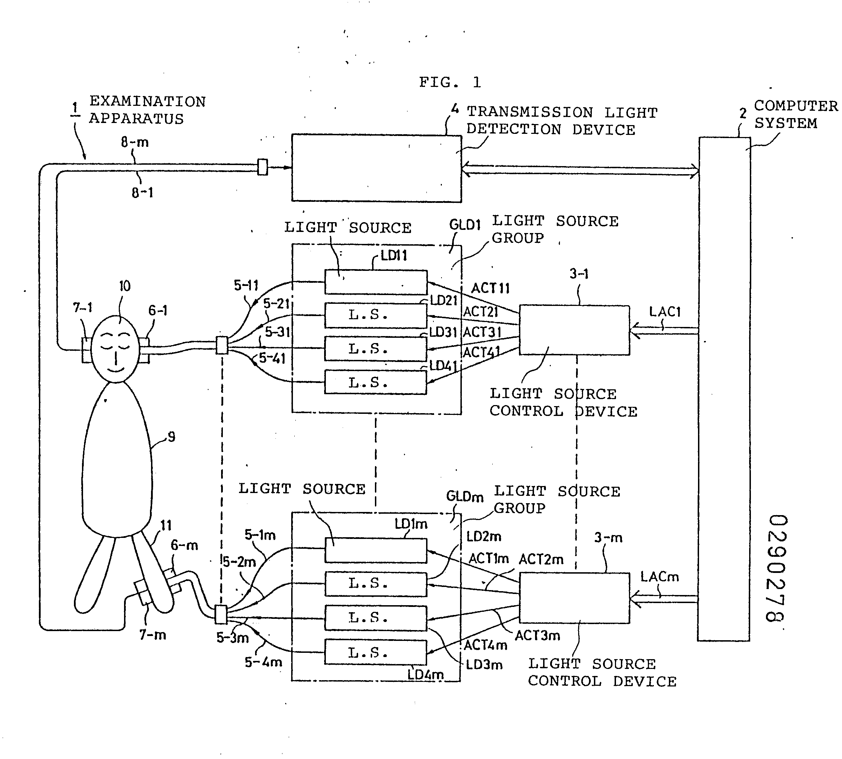

Fig. 1 shows a system constitution of an examination apparatus according to the first embodiment of the invention. In the examination apparatus shown in Fig. 1, plural light source control devices 3-1 to 3-m and one transmission light detection device 4 are connected to a computer system 2. Each of light source groups GLD1 to GLDm is connected to and driven by the corresponding one of light source control devices 3-1 to 3-m. The light source group GLD1 is equipped with four light sources LD11, LD21, LD31 and LD41 which emit four near infrared light rays with respective wavelengths $\lambda_1$, $\lambda_2$, $\lambda_3$, and $\lambda_4$. In the same manner, the light source group GLDm is equipped with four light sources LD$_{1m}$, LD$_{2m}$, LD$_{3m}$ and LD$_{4m}$ which emit four near infrared light rays with respective wavelengths $\lambda_1$, $\lambda_2$, $\lambda_3$, and $\lambda_4$.

Optical fibers 5-11 to 5-41 are connected to the respective light sources LD11 to LD41 in the light source group GLD1 to introduce emitted near infrared light rays. Ends of the optical fibers are held by an illumination-side fixture 6-1. In the same manner, optical fibers 5-1m to 5-4m are connected to the respective light sources LD1m to LD4m in the light source group GLDm and ends of the optical fibers 5-1m to 5-4m are held by an illumination-side fixture 6-m. The examination apparatus 1 is equipped with the plural light source groups GLD1 to GLDm and the plural illumination-side fixtures 6-1 to 6-m, one of the illumination fixtures corresponding to one of the light source groups. Plural detection-side fixtures 7-1 to 7-m correspond to the respective illumination-side fixtures 6-1 to 6-m. Optical fibers 8-1 to 8-m are held by the respective detection-side fixtures 7-1 to 7-m and are connected to a common transmission light detection device.

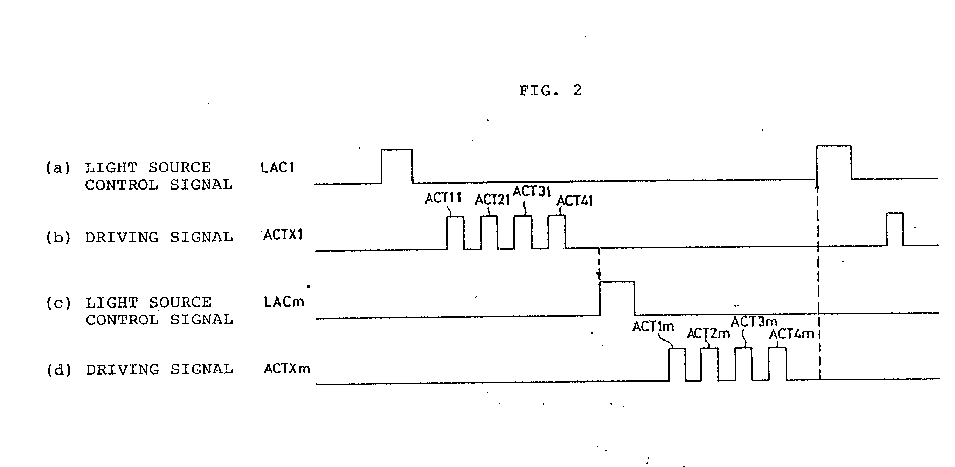

In the examination apparatus with the foregoing constitution, the computer system 2 gives a light source control signal LAC1 to the light source control device 3-1 with a timing shown in Fig. 2(a) and also gives a light source control signal LACm to the light source control device 3-m with a timing shown in Fig. 2(c). In the time-charts of Fig. 2(a) to (d), assuming that there equipped are two light source control devices, two light source groups, two illumination-side fixtures and two detection-side fixtures, "m" is selected to be "2". When the light source control signal LAC1 is given to the light source control device 3-1, the light source control device 3-1 sequentially sends driving signals ACT11 to ACT41 to the respective light sources LD11 to LD41 as shown in Fig. 2(b). Responding to these driving signals, the near infrared light rays with different wavelengths $\lambda_1$ to $\lambda_2$ are emitted from the respective light sources LD11 to LD41 in the light source group GLD1 and are made incident on for example a head 10 of an object person 9. Transmission light rays from the head 10 are sent to the transmission light detection device 4 and transmission quantities $t_{\lambda 1}$, $T_{\lambda 2}$, $T_{\lambda 3}$ and $T_{\lambda 4}$ are counted by the transmission light detection device 4.

After making the driving signals ACT11 to ACT41 be sequentially outputted according to the light source control signal LAC1, sequentially driving light sources LD11 to LD41 in the light source group GLD1 and counting the transmission quantities $T_{\lambda 1}$, $T_{\lambda 2}$, $T_{\lambda 3}$ and $T_{\lambda 4}$ of the near infrared light rays transmitted from the head 10, the computer system 2 sends the light control signal LACm to the light source control device 3-m as shown in Fig. 2(c). When the light source control signal LACm is given to the light source control device 3-m, the light source control device 3-m sequentially sends driving signals ACT1m to ACT4m to the respective light sources LDm1 to LD4m as shown in Fig. 2(d). Responding to these driving signals, the near infrared light rays with different wavelengths $\lambda_1$ to $\lambda_4$ are sequentially emitted from the respective light sources LD1m to LD4m in the light source group GLDm and are made incident on for example a part of a leg 11 of the object person 9. Transmission light rays from the leg 11 are sent to the transmission light detection device 4 and the transmission quantities $T_{\lambda 1}'$, $T_{\lambda 2}'$, $T_{\lambda 3}'$ and $T_{\lambda 4}'$ are counted by the transmission light detection device 4. By giving the light source control signals LAC1 and LACm alternately to the respective light source control devices 3-1 and 3-m from the computer system 2 so as to alternately illuminate the head 10 and the leg 11 with a time-sharing method, the transmission quantities $T_{\lambda 1}$, $T_{\lambda 2}$, $T_{\lambda 3}$ and $T_{\lambda 4}$ and the transmission quantities $T_{\lambda 1}'$, $T_{\lambda 2}'$, $T_{\lambda 3}'$ and $T_{\lambda 4}'$ are alternately counted over a predetermined measuring period so that transmission quantity data $T_{\lambda 1}$, $T_{\lambda 2}$, $T_{\lambda 3}$ and $T_{\lambda 4}$ and transmission quantity data $T_{\lambda 1}'$, $T_{\lambda 2}'$, $T_{\lambda 3}'$ and $T_{\lambda 4}'$ are obtained.

In the foregoing examination apparatus according to the first embodiment of the present invention, there equipped are the one computer system 2, the plural light source control devices 3-1 to 3-m, the plural light source groups GLD1 to GLDm, the plural illumination-side fixtures 6-1 to 6-m and the plural detection-side fixtures 7-1 to 7-m, and the transmission quantities of the near infrared light rays transmitted from the plural parts of an object person are alternately measured with a time-sharing method. Therefore, the efficiency of use of the computer system 2 can be greatly improved.

Though in the foregoing description it is assumed that the plural light source control devices 3-1 to 3-m drive the respective light source groups GLD1 to GLDm, the examination apparatus of the invention may be constituted with only one light source control device for driving the plural light source groups GLD1 to GLDm. Moreover, though in the foregoing description it is assumed that the plural parts of one object person are measured with a time-sharing method, for example the same part of plural object persons may be measured with a time-sharing method with the same constitution as in the foregoing.

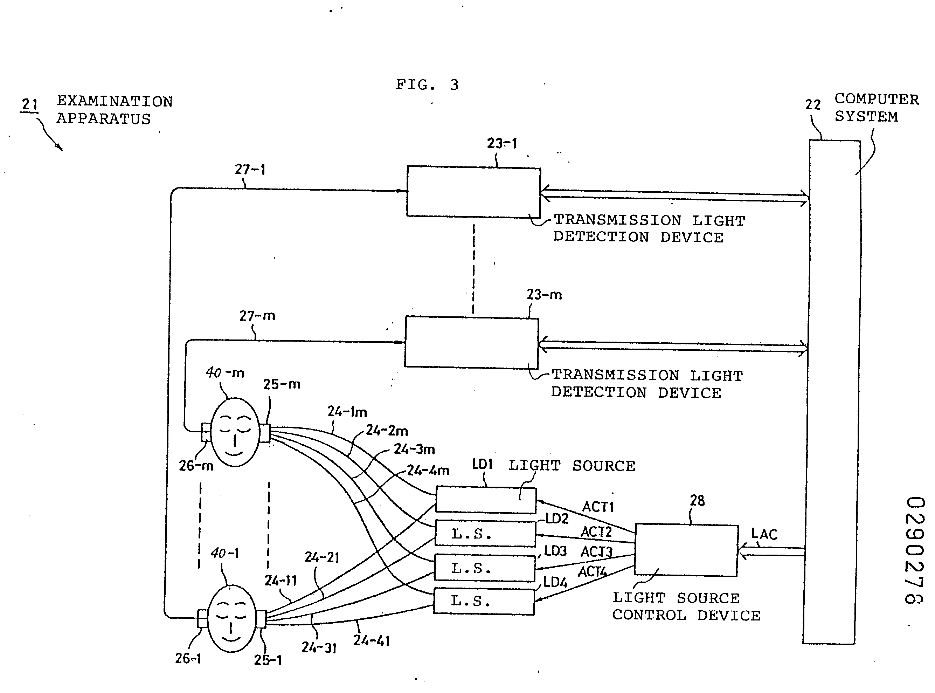

Fig. 3 shows a system constitution of an examination apparatus according to the second embodiment of the present invention. In an examination apparatus 21, one light source control device 28 and plural transmission light detection devices 23-1 to 23-m are connected to a computer system 22. The light source control device 28 sequentially drives plural light sources LD1 to LD4. After the light source LD1 has been driven and transmission quantities have been counted by the transmission light detection devices 23-1 to 23-m, the light source LD2 is driven. To each of the light sources LD1 to LD4 is connected one corresponding group of optical fiber groups which have optical fibers 23-11 to 24-1m, 24-21 to 24-2m, 24-31 to 24-3m and 24-41 to 24-4m, respectively. Ends of the optical fibers 24-11 to 24-41 through ends of the optical fibers 24-1m to 24-4m are bundled and held by illumination-side fixtures 25-1 through 25-m, respectively. Plural detection-side fixtures 25-1 to 25-m. Ends of optical fibers 27-1 to 27-m are held by the respective detection-side fixtures 26-1 to 26-m. The optical fibers 27-1 to 27-m are connected to the respective transmission light detection devices 23-1 to 23-m.

In the examination apparatus 21, when the computer system 22 gives a light source control signal LAC to the light source control device 28, the light source control device 28 firstly drives the light source LD1 through a driving signal ACT1 so that the light source LD1 emits near infrared light with wavelength $\lambda_1$. The near infrared light with the wavelength $\lambda_1$ emitted from the light source LD1 is divided and introduced to for example object persons, heads 40-1 to 40-m by the respective optical fibers 24-11 to 24-1m. Therefore, the heads 40-1 to 40-m are simultaneously illuminated by the near infrared light rays with the wavelength $\lambda_1$ emitted from the light source LD1. Transmitted near infrared light rays with the wavelength $\lambda_1$ from the heads 40-1 to 40-m are sent to the respective transmission light detection devices 23-1 to 23-m substantially at the same time and thereby the transmission quantities from the heads 40-1 to 40-m are counted. The transmission light detection devices 23-1 to 23-m temporarily store counted data of the transmission quantities and sequentially send counted data corresponding to the transmitted near infrared light rays with the wavelength $\lambda_1$ from the heads

40-1 to 40-m to the computer system 22 in a time-sharing method. After these procedures the computer system 22 gives the second light source control signal LAC to the light source control device 28. The light source control device 28 drives the light source LD2 through a driving signal ACT2 so that the light source LD2 emits near infrared light with wavelength $\lambda_2$. In the same manner as the above-described procedure, the near infrared light rays with the wavelength $\lambda_2$ from the heads 40-1 to 40-m are sent to the respective transmission light detection devices 23-1 to 23-m substantially at the same time and thereby the transmission quantities from the heads 40-1 to 40-m are counted. Counted data of the transmission quantities from the heads 40-1 to 40-m are sequentially sent to the computer system 22 with the time-sharing method.

In the same manner, the light source LD3 is driven, transmission quantities of near infrared light rays with wavelength $\lambda_3$ from the heads 40-1 to 40-m are counted and counted data of the transmission quantities are sequentially sent to the computer system 22. Then, the light source LD4 is driven and counted data of transmission quantities of near infrared light rays with wavelength $\lambda_4$ from the heads 40-1 to 40-m are sequentially sent to the computer system 22. The plural object persons' heads can be examined by the foregoing procedure.

In the foregoing examination apparatus according to the second embodiment of the invention, the plural optical fibers are connected to each of light sources, for example the heads 40-1 to 40-m of the plural object persons are simultaneously illuminated and the transmission quantities from the heads 40-1 to 40-m are counted by the respective plural transmission light detection devices 23-1 to 23-m. Therefore, the system efficiency of the computer system 22 can be improved.

Though in the foregoing description of the second embodiment it is assumed that the same part, for example the head, of the plural object persons are examined, this embodiment may be adapted to the examination of plural parts of the one object person.

Though each light source group has plural light sources in the foregoing description of the embodiments, the electromagnetic waves with different wavelengths may be obtained by filtering a white light emitted from one white light source. And the electromagnetic wave emitted from the light source is not limited to near infrared light, that is, far infrared light, visible light, microwave, etc. may be used.

Moreover, the application of the examination apparatus according to the present invention is not limited to the medical field but covers many fields including mere measurements. And measuring objects are not limited to object persons but may be general objects.

## Claims

1. An examination apparatus for measuring the oxygenation of objects by electromagnetic wave transmission spectrophotometry, comprising:

light source means (LD1m-LD4M, 3-1 to 3-m) for sequentially emitting electromagnetic radiation at a number of different wavelengths;

transmitted light detection means (4) for detecting electromagnetic radiation transmitted through an object and outputting transmission light data; and,

a computer system (2) for controlling the light source means (LD1m-LD4m, 3-1 to 3-m) and the transmitted light detection means (4) and for receiving the transmission light data from the transmission light detection means (4) and calculating the oxygenation parameters;

characterised in that the examination apparatus also includes:

a plurality of illumination-side fixtures (6-1, ... 6-m) for applying electromagnetic radiation emitted by the light source means (LD1m-LD4m) to a plurality of measurement locations; and,

a plurality of detection-side fixtures (7-1, ... 7-m) for receiving electromagnetic radiation transmitted through the plurality of measurement locations and coupling the transmitted electromagnetic radiation to the transmitted light detection means;

the computer system (2) calculating the oxygenation parameters for each of the plurality of measurement locations.

2. An examination apparatus as claimed in claim 1, wherein the plurality of measruement locations are a plurality of parts of the same object, person or animal.

3. An examination apparatus as claimed in claim 1, wherein the plurality of measurement locations are located on a plurality of objects, persons or animals.

4. An examination apparatus as claimed in any one of the preceding claims, wherein the light source means comprises a plurality of light source groups (GLD1-GLDm), each light source group being connected to a corresponding one of the plurality of illumination-side fixtures (6-1 to 6-m); and,

the transmitted light detection measn comprises a single common transmission light detection device (4) connected to the plurality of detection-side fixtures (7-1 to 7-m).

5. An examination apparatus as claimed in claim 4, wherein the plurality of light source groups (GLD1-GLDm) are driven by a corresponding plurality of light source control means (3-1 to 3-m) in accordance with instructions sent from the computer system (2).

6. An examination apparatus as claimed in claim 4, wherein the plurality of light source groups (GLD1-GLDm) are driven by a single common light source control means (3) in accordance with instructions sent from the computer system (2).

7. An examination apparatus as claimed in any one of claims 4, 5 or 6; wherein the plurality of measurement locations are illuminated by the electromagnetic radiation successively introduced by the plurality of light source groups (GLD1-GLDm) in a manner such that a second light source group emits the elecromagnetic radiation after a first light source group has finished emitting the electromagnetic radiation of all of the different wavelengths.

8. An examination apparatus as claimed in any one of claims 1 to 3, wherein the light source means comprises one common light source group (LD1-LD4) connected to the plurality of illumination-side fixtures (25-1 to 25-m); and,

the transmission light detection means comprises a plurality of transmission light detection devices (23-1 to 23-m), the plurality of transmission light detection devices (23-1 to 23-m) being connected to corresponding ones of the detection-side fixtures (26-1 to 26-m).

9. An examination apparatus as claimed in claim 8, wherein the plurality of measurement locations are illuminated by respective electromagnetic radiation of the same wavelength substantially at the same time; and,

the plurality of transmission light detection devices (23-1 to 23-m) temporarily store their respective transmission light data and sequentially send the respective transmission light data with a time-sharing method to the computer system (22).

FIG. 1

FIG. 2

(a) LIGHT SOURCE    LAC1
    CONTROL SIGNAL

(b) DRIVING SIGNAL  ACTX1
    ACT11  ACT21  ACT31  ACT41

(c) LIGHT SOURCE    LACm
    CONTROL SIGNAL

(d) DRIVING SIGNAL  ACTXm
    ACT1m  ACT2m  ACT3m  ACT4m

0290278

FIG. 3

21 EXAMINATION APPARATUS

22 COMPUTER SYSTEM

23-1

27-1

TRANSMISSION LIGHT DETECTION DEVICE

23-m

27-m

TRANSMISSION LIGHT DETECTION DEVICE

40-m  25-m

24-1m
24-2m
24-3m
24-4m

26-m

LD1  LIGHT SOURCE

ACT1  28

LD2  ACT2  LAC

L.S.

LD3  ACT3

40-1  L.S.

24-21  LD4  ACT4

24-11  L.S.

26-1  25-1  24-31  24-41

LIGHT SOURCE CONTROL DEVICE

0290278

FIG. 4(a)

FIG. 4(b)

0290278

FIG. 5

TRANSMISSION LIGHT
DETECTION DEVICE 54

TEMP. CONTROLLER

COMPUTER SYSTEM 56

CPU 62

DETECTION CONTROLLER

68

45 EXAMINATION APPARATUS

57 FILTER

53

67

59 60 CTL 61

PMT AMP

MEMORY 63

70 71 69 58

AMPLITUDE DISCRIMINATOR

MULTI-CH. PHOTON-COUNTER

OUTPUT DEVICE 64

INPUT DEVICE 65

52

40

51

66 SYSTEM BUS

LIGHT SOURCE

LIGHT SOURCE. CONTROL DEVICE

50-1 LD1

50-2 L.S. LD2 ACT1

50-3 L.S. LD3 ACT2

50-4 L.S. LD4 ACT3

55

ACT4

0290278

FIG. 6

(a) DRIVING SIGNAL ACT1

(b) DRIVING SIGNAL ACT2

(c) DRIVING SIGNAL ACT3

(d) DRIVING SIGNAL ACT4

(e) CONTROL SIGNAL CTL

$\phi11$ $\phi12$ $\phi13$ $\phi14$ $\phi15$   $\phi n1$ $\phi n2$ $\phi n3$ $\phi n4$ $\phi n5$   $\phi N1$ $\phi N2$ $\phi N3$ $\phi N4$ $\phi N5$

CY1   CYn   CYN

Mk

0290278

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | US-A-4 281 645 (F.F. JÖBSIS) <br> * Column 1, lines 10-20; column 5, lines 14-43; column 13, line 38 - column 14, line 6; column 18, line 65 - column 19, line 52; figures 5,6,10,11 * | 1,4,5,7 | A 61 B 5/00 <br> G 01 N 21/31 |
| A | | 2 | |
| Y | JP-A-60 232 132 (SNLE-STANLEY ELEC.) <br> * Whole document * & DERWENT WPIL ABSTRACT | 1,4,5,7 | |
| A | | 3,9 | |
| A | GB-A-2 162 939 (SCLAVO S.p.A.) <br> * Abstract; page 4, lines 32-58; figure 8 * | 1,2,6,8 ,9 | |
| A | US-A-4 509 522 (T.J. MANNUCCIA et al.) <br> * Column 2, lines 15-20; column 3, lines 1-6,11-14; column 6, line 45 - column 7, line 2; figure 4 * | 1,3 | |
| A | US-A-4 183 360 (W. CARLSON et al.) <br> * Abstract; column 2, line 62 - column 3, line 5; figure 1 * | 2,6,8 | TECHNICAL FIELDS SEARCHED (Int. Cl.4) <br><br> A 61 B <br> G 01 N |
| A | MEDICAL & BIOLOGICAL ENGINEERING & COMPUTING, vol. 21, no. 2, March 1983, pages 229-231, IFMBE, Stevenage, Herts, GB; R. VAN MASTRIGT et al.: "Analogue data collection via a telephone line: a microprocessor-based remote-controlled data-acquisition system" <br> * Whole article * | 9 | |

The present search report has been drawn up for all claims

| Place of search | Date of completion of the search | Examiner |
|---|---|---|
| THE HAGUE | 19-07-1988 | FERRIGNO, A. |

EPO FORM 1503 03.82 (P0401)